# EUROPEAN PATENT SPECIFICATION

(11) **EP 1 627 423 B1**
(45) Date of publication and mention of the grant of the patent: **25.01.2012**
(21) Application number: 04722621.2
(22) Date of filing: 23.03.2004
(51) Int. Cl.: A61B 17/02, A61B 17/28

(54) **SURGICAL HOLDER FOR A BLOOD VESSEL**
CHIRURGISCHES HALTEINSTRUMENT FÜR EIN BLUTGEFÄSS
DISPOSITIF CHIRURGICAL DE MAINTIEN POUR VAISSEAU SANGUIN

(30) Priority: 25.03.2003 JP 2003082438; 25.09.2003 JP 2003334379
(43) Date of publication of application: 22.02.2006
(73) Proprietor: Cardio Incorporated, Osaka-shi, Osaka 530-0043 (JP); Watanabe, Go, Ishikawa 921-8221 (JP)
(72) Inventor: WATANABE, Go, Ishikawa 921-8221 (JP); SAWA, Yoshiki, Hyogo 662-0099 (JP); TAKETANI, Satoshi, Osaka 534-0027 (JP)
(74) Representative: Harding, Charles Thomas
(86) International application number: PCT/IB2004/050314
(87) International publication number: WO 2004/086490

(56) References cited:
- US-A- 4 286 598
- US-A- 5 496 341
- US-A- 6 106 542

## Description

TECHNICAL FIELD

The present invention relates to a surgical holder, particularly, a surgical holder for blood vessel surgery which is suitable for coronary bypass surgery and the like.

BACKGROUND ART

Conventionally, bypass surgery using heart-lung machines has been generally conducted for patients suffering myocardial infarction or angina pectoris. When such surgery is conducted, extracorporeal circulation accompanied with the use of heart-lung machines causes complication to occur at a certain ratio, and affects prognosis of the patient.

In recent years, surgical operations have increasingly become less invasive. In the field of cardiac surgery, bypass surgery on a beating heart which does not employ heart-lung machines has become widely used. In bypass surgery on a beating heart, while a removed artery or vein is being connected to the heart, generally, an assistant holds part of a blood vessel of the patient with tweezers.

Grasping a tissue such as a blood vessel with tweezers requires skill. When a very delicate tissue is grasped, the tissue may be undesirably damaged. Further, there is a problem that a sufficient view is required for manipulation in surgery, the view may be obstructed by an operation of holding a blood vessel. Conventionally, a tool for grasping a blood vessel has been developed (see, Japanese Utility Model Laid-Open Publication No. 7-17208, US-A-5 496 341 and US-A-4 286 598), but further improvement is demanded in terms of manipulability, stability in grasping, applicability, and the like. Furthermore, a tool which may be used for manipulating the grasped tissue such as during anastomosis manipulation, a tool having a multifunction which may by itself address various manipulations during surgery, and the like has been strongly desired.
The objective of the present invention is to provide a tool which can stably grasp a tissue such as a blood vessel without causing damage and which enables various manipulations during surgery.
In view of the problems of the conventional art, the present inventors have conducted a diligent study. As a result, the present inventors have found that a tool having a specific structure well adapted to manipulations during surgery, and achieved the present invention through further study.

### DISCLOSURE OF THE INVENTION

The present invention is defined in claim1. Preferred embodiments are defined in the dependent claims.

The surgical holder of the present invention is preferably a surgical holder for blood vessel surgery, and more preferably, a surgical holder for coronary bypass surgery.

Furthermore, the surgical holder of the present invention is preferably a surgical holder in which a tubular tissue to be grasped is a blood vessel.

The surgical holder of the present invention can grasp and fix tissues of various forms and can preferably grasp blood vessel tissues and the like including almost all the types of artery and vein. For example, it is possible to grasp a tissue removed with a surrounding tissue thereof or to grasp a skeletonized tissue.

Furthermore, the skeletonized tissue can be grasped at a plurality of points at one time. Moreover, a further manipulation on the grasped tissue such as a sequential grafting or the like can be performed. The first grasping plate and the second grasping plate can be used as a supporting platform or a manipulation platform to improve stability of manipulation.

Further, the surgical holder of the present invention may have a tissue protection material 10 having a slip stopping function and a tissue protection function. Thus, a tissue can be stably grasped without damaging the tissue.

Further, it is possible to permeate the tissue protection material with medicines and appropriately perform treatments such as preventing a tissue from being dried or preventing cell injury.

Further, the holder may have wiring portion, and it is possible to freely set the position of a grasping member 1 and a manipulation member 2. Moreover, it is possible to enhance convenience of an operator by fixing another end of the wiring portion to a rib spreader or the like.

Conventionally, a tissue was held by an assistant or the like during surgery. According to the holder of the present invention, someone's assistance in grasping is not required. Moreover, the problem that a person's hand obstructs the view can be solved.

Furthermore, since the holder of the present invention has a compact structure, a cut portion in surgery can be made smaller, and thus, damage on a body of a patient can be reduced. The holder of the present invention has a simple structure and can be made of a widely used material such as a resin. Thus, a cost can also be reduced.

Moreover, the holder of the present invention is a multifunctional tool which can address various operations by itself. Thus, there is no need to use a plurality of tools and surgical manipulations become more convenient and efficient.

The present invention with the above-described advantages has particularly superior effects in less invasive surgery, of which the number of applied cases is predicted to increase, and thus, is useful.

BRIEF DESCRIPTION OF THE DRAWINGS

Figure **1** shows an exemplary grasping member, manipulation member, connection portion and wiring portion of the holder of the present invention;

Figure **2** is a plan view of an exemplary grasping member and manipulation member of the holder of the present invention when viewed from the side of a first grasping plate;

Figure **3** is a plan view of an exemplary grasping member and manipulation member of the holder of the present invention when viewed from the side of a second grasping plate;

Figure **4** is a schematic view of a part of the manipulation of the holder of the present invention with arrows indicating a direction of force applied to the manipulation member and a direction of movement of the grasping member in cooperation with the movement of the manipulation member;

Figure **5** is a view of an exemplary grasping member and the manipulation member of the holder of the present invention when viewed from the side of the opening;

Figure **6** is a view of an exemplary grasping member and the manipulation member of the holder of the present invention when viewed from the side of a recessed portion of the first grasping plate and a curved portion of the second grasping plate;

Figure **7** is a schematic view of a part of the manipulation of the holder of the present invention when viewed from the side with arrows indicating a direction of force applied to the manipulation member and a direction of movement of the grasping member in cooperation with the movement of the manipulation member;

Figure **8** is a schematic view showing a tubular tissue being grasped with a surrounding portion thereof by the holder of the present invention;

Figure **9** is a schematic view showing a skeletonized tubular tissue being grasped by the holder of the present invention;

Figure **10** is a schematic view showing a skeletonized tubular tissue being grasped at two points by the holder of the present invention;

Figure **11** is a schematic view showing a tubular tissue being grasped by the holder of the present invention;

Figure **12** is a schematic view showing sequential grafting being performed by using the holder of the present invention;

Figure **13** is a plan view of another exemplary grasping member and manipulation member of the holder of the present invention when viewed from the side of a first grasping plate;

Figure **14** is a plan view of another exemplary grasping member and manipulation member of the holder of the present invention when viewed from the side of a second grasping plate;

Figure **15** is a plan view of yet another exemplary grasping member and manipulation member of the holder of the present invention when viewed from the side of a first grasping plate;

Figure **16** is a plan view of yet another exemplary grasping member and manipulation member of the holder of the present invention when viewed from the side of a second grasping plate;

Figure **17** is a plan view of still another exemplary grasping member and manipulation member of the holder of the present invention when viewed from the side of a first grasping plate;

Figure **18** is a plan view of still another exemplary grasping member and manipulation member of the holder of the present invention when viewed from the side of a second grasping plate; and

Figures **19A** through **19E** are schematic views of various shapes of a tissue grasping space, not according to the present invention, formed between the first grasping plate and the second grasping plate.

BEST MODE FOR CARRYING OUT THE INVENTION

The embodiments of the present invention will be specifically described below.

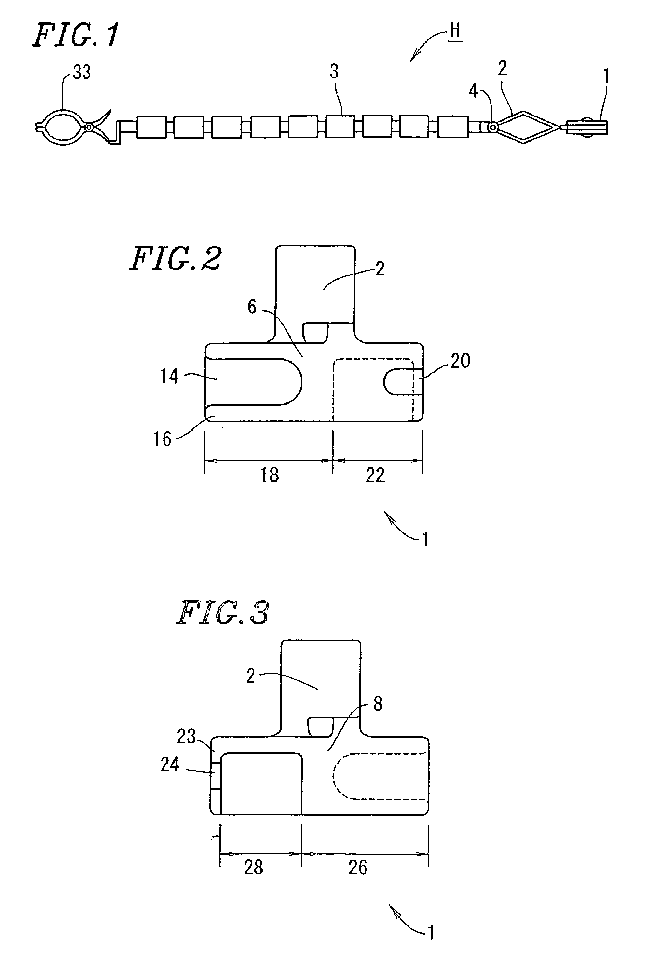

As shown in Figure **1**, a surgical holder H according to the present invention includes a grasping member **1** for grasping a tissue, manipulation member **2** for manipulating the grasping member **1**, and a wiring portion **3** connected to the manipulation member **2**, which is a coupling portion. The holder H according to the present invention may further include a connection portion **4** for connecting the wiring portion **3** and the manipulation member **2.**

(Grasping Member **1**)

The grasping member **1** is a part for grasping a tissue and includes a first grasping plate **6** and a second grasping plate **8.**

The first grasping plate **6** and the second grasping plate **8** can be overlaid and oppose each other. A tissue inserted between the first grasping plate **6** and the second grasping plate **8** is pinched and grasped with opposing inner side surfaces of the first grasping plate **6** and the second grasping plate **8.** More specifically, the second grasping plate **8** is provided so as to oppose the first grasping plate **6** in a movable manner so that they are able to become closer to each other or more distanced from each other.

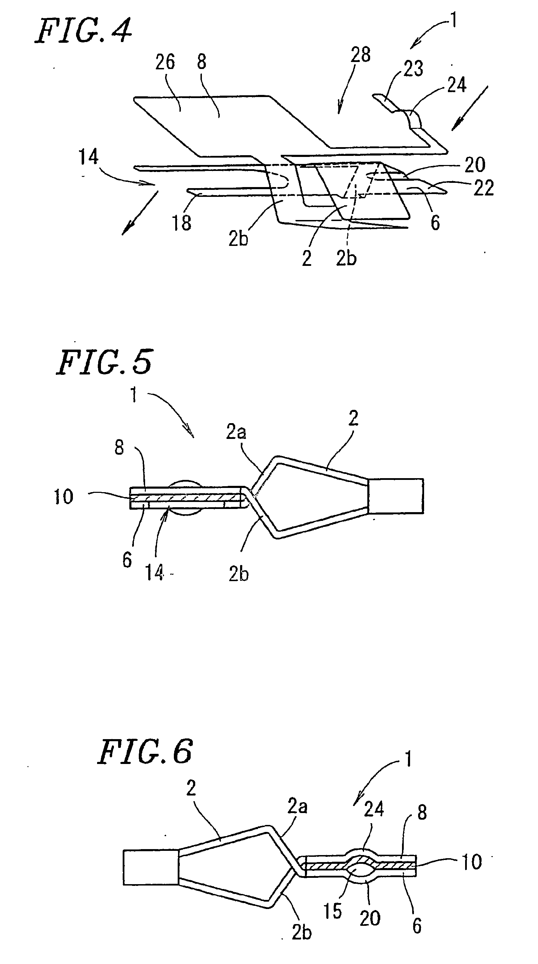

As shown in Figure **2** through **7**, the first grasping plate **6** includes a retaining portion **18** having an opening **14** of a U-shape or substantially a U-shape, and a supporting portion **22** having a recessed portion **20.** The peripheral portion of the retaining portion **18** except for the opening **14** and the supporting portion **22** are continuous.

The retaining portion **18** of the first grasping plate **6** pinches a tissue with a covering portion **26** of the second grasping plate **8** and retains the tissue. The retaining portion **18** has the opening **14** of a U-shape or substantially a U-shape and an edge portion **16** which defines the shape of the opening **14.**

The opening **14** is a notch portion of a U-shape or substantially a U-shape which is located in an end portion of the first grasping plate **6**. The term 'substantially a U-shape' means a shape approximately the same as a U-shape and includes, for example, a semiellipse shape, and a semi-quadrilateral shape. The edge portion **16** which defines the shape of the opening **14** is the peripheral portion of the opening **14.** End portions of the edge portion **16** which defines the shape of the opening **14** define upper edges of the 'u' in the U-shape or substantially U-shape of the opening **14** and are elongated.

In other words, a first grasping portion which can grasp a part of a tissue between the first grasping plate **6** and the second grasping plate 3 and the opening **14.** which may expose another part of the tissue when the part of the tissue is grasped by the first grasping portion are provided in one end portion of the grasping member **1.** Further, a second grasping portion which may form a tissue grasping space **15** between the first grasping plate **6** and the second grasping plate 8 is provided in the other end portion of the grasping member **1.** The first grasping portion includes the retaining portion **18** of the first grasping plate **6** and the covering portion **26** of the second grasping plate **8.** The second grasping portion includes the recessed portion **20** of the first grasping plate **6** and a curved portion **24** of the second grasping plate **8.**

The above-described structure enables an appropriate grasping of a tissue. For example, if a tissue to be grasped is a tubular tissue and a surrounding tissue thereof, the surrounding tissue is pinched by the retaining portion **18** which defines the shape of the opening **14** of the first grasping plate **6** and the covering portion **26** of the second grasping plate **8** to position the tubular tissue at the opening **14.** Thus, it is possible to grasp a surrounding tissue without directly retaining a tubular tissue which is susceptible to damage. Further, if a tissue to be grasped is a tubular tissue which has a stump, the end portion of the edge portion **16** of the retaining portion **18** which defines the shape of the opening **14** of the first grasping plate **6** is inserted into a tube of the tubular tissue to grasp the tubular tissue so the tissue hangs suspended from a tip portion of the edge portion **16**.

The supporting portion **22** of the first grasping plate **6** supports the tissue being grasped, and has the recessed portion **20** in the end portion of the supporting portion **22**. The recessed portion **20** of the supporting portion **22** and the opening **14** of the retaining portion **18** are preferably positioned at both ends of one straight line. The recessed portion **20** forms a pair with the curved portion **24** of the second grasping plate **8** to form the tissue grasping space **15**. A shape of the recessed portion **20** is not specifically limited. However, the recessed portion **20** is preferably a recess of a semicircle.

In addition to the retaining portion **18**, the supporting portion **22** also enables appropriate grasping of a tissue. For example, if a tissue to be grasped is a tubular tissue, the tubular tissue is grasped by the retaining portion **18** of the first grasping plate **6** and the covering portion **26** of the second grasping plate **8** at one point, and the tubular tissue is grasped by the tissue grasping space **15** formed by the recessed portion **20** of the first grasping plate **6** and the curved portion **24** of the second grasping plate **8** at another point. By grasping the tubular tissue at a plurality of points, it is possible to grasp the tissue more stably.

Furthermore, the supporting portion **22** may be used as a manipulation platform for further manipulation or processing of the grasped tissue. For example, in the case where the tubular tissue is grasped at at least two points, the tubular tissue between the two points is positioned on the supporting portion **22**. Thus, manipulation such as anastomosis manipulation or the like can be performed on the supporting portion **22**.

A tissue protection material **10** to be attached to the first grasping plate **6** and/or the second grasping plate 8 is formed so as to position between the first grasping plate **6** and the second grasping plate **8.** A range to be covered by tissue protection material **10** may be an entire surface or a part of the first grasping plate **6** and/or the second grasping plate **8**. However, the tissue protection material **10** is preferably attached to the entire surface on the side of the second grasping plate **8** which opposes the first grasping plate **6**.

The tissue protection material **10** has both a tissue protection function and a slip stopping function. The tissue protection material **10** allows the prevention of damage to the tissue to be grasped and a more stable grasping of the tissue. The tissue protection material **10** may be permeated with various medicines, or subjected to a surface process to give additional functions to the surgical holder H.

The tissue protection material **10** is preferably flexible, and, for example, sponge or the like may be suitably used. The type of base material used for the tissue protection material **10** is not particularly limited. However, it is preferable to be a hydrophilic base material. If a hydrophilic base material is used, it becomes easy to have the tissue protection material **10** permeated with a solution including various medicines to give functions such as preventing a tissue from being dried or preventing cell injury. The medicines permeating through the tissue protection material **10** may be, for example, an anticoagulant agent, a vasodilator, a blood vessel growth promotion factor, various proteins, an immunosuppressive agent, and the like.

The second grasping plate **8** includes a covering portion **26** which is formed to cover an entire surface or a part of the opening **14** of the first grasping plate **6**, a non-covering portion **28** which does not cover the first grasping plate **6**, and a fixing portion **23** having the curved portion **24.** More specifically, the second grasping plate 8 includes the fixing portion **23** provided to extend from the covering portion **26,** which is formed so as to cover an entire surface or a part of the opening **14** of the first grasping plate **6**, through an elongated piece, and includes the non-covering portion **28** with an opening of a rectangular shape formed by an edge of the covering portion **26**, the elongated piece, and the fixing portion **23.**

The covering portion **26** of the second grasping plate **8** covers an entire surface or a part of the opening **14** of the first grasping plate 6, when the first grasping plate 6 and the second grasping plate **8** are manipulated so as to oppose each other and overlay. It is preferable that the covering portion **26** is formed so as to cover the entire surface of the opening **14**, and it is further preferable that the covering portion **26** is formed so as to cover not only the opening **14**, but also surrounding portions of the opening **14**. Particularly, when the first grasping plate **6** and the second grasping plate **8** are manipulated so as to oppose each other and overlay, it is preferable that the covering portion **26** is formed so as to cover the retaining portion **18** of the first grasping plate **6**.

The covering portion **26** pinches a tissue with the retaining portion **18** of the first grasping plate **6** to grasp the tissue. Further, the covering portion **26** supports or fixes the grasped tissue to improve the stability. For example, if a tissue to be grasped is a tubular tissue and the surrounding tissue thereof and the surrounding tissue is pinched by the retaining portion **18** which defines the shape of the opening **14** of the first grasping plate **6** and the covering portion **26** of the second grasping plate **8**, the tubular tissue positioned at the opening **14** is supported by the covering portion **26**. Further, if a tissue to be grasped is a tubular tissue which has a stump and the end portion of the edge portion **16** of the retaining portion **18** which defines the shape of the opening **14** of the first grasping plate **6** is inserted into a tube of the tubular tissue to grasp the tubular tissue so the tissue hangs suspended from a tip portion of the edge portion **16,** the tubular tissue can be grasped more stably by pressing the side surface of the tubular tissue grasped by the covering portion **26.**

The non-covering portion **28** of the second grasping plate **8** does not cover the first grasping plate **6** when the first grasping plate 6 and the second grasping plate **8** are manipulated so as to oppose each other and overlay. The non-covering portion **28** approximately corresponds to the supporting portion **22** of the first grasping plate 6 when the first grasping plate **6** and the second grasping plate **8** oppose each other.

The non-covering portion **28** enables a manipulation or process with the surgical holder H grasping a tissue. For example, when the retaining portion **18** of the first grasping plate **6** and the covering portion **26** of the second grasping plate 8 grasp a tubular tissue at one point, the tissue grasping space **15** which is formed with the recessed portion **20** of the first grasping plate 6 and the curved portion **24** of the second grasping plate **8** grasps the tubular tissue at another point, and a middle portion of the tubular tissue between the two points is positioned on the supporting portion **22,** it becomes possible to appropriately perform manipulation or processing of the tissue on the supporting portion **22** from the side of the opening of the non-covering portion **28.**

The fixing portion **23** of the second grasping plate **8** has a curved portion **24** and fixes the tissue. The fixing portion **23** and the covering portion **26** are preferably positioned at respective ends on one axis of the second grasping plate **8** with the non-covering portion **28** being therebetween.

The curved portion **24** opposes the recessed portion **20** of the first grasping plate **6** to form the tissue grasping space **15** when the first grasping plate 6 and the second grasping plate **8** are positioned so as to oppose one another. The shape of the tissue grasping space **15** forms substantially circular space **15**. The tissue grasping space **15** enables appropriate grasping of, for example, a skeletonized tubular tissue without cutting it.

The material to form the first grasping plate **6** and the second grasping plate **8** is not specifically limited. Various types of polymer, such as polypropylene, acrylonitrile butadiene styrene copolymer (ABS), polymethyl pentene, and the like may be used. The first grasping plate **6** and the second grasping plate **8** may be transparent substrates.

The size of the first grasping plate **6** and the second grasping plate **8** is not specifically limited and can be appropriately set depending on the types of tissue to be grasped. For example, if the tissue to be grasped is a blood vessel, the grasping member 1 may have a length of about 20 to 40 mm and a width of about 10 to 20 mm, and preferably, a length of about 25 to 35 mm and a width of about 12 to 15 mm.

(Manipulation Member **2**)

The manipulation member **2** performs a manipulation of the grasping member **1** in a movable manner so that plates of the grasping member **1** are able to become closer to each other or more distanced from each other. More specifically, the manipulation member **2** performs manipulation to move the first grasping plate **6** and the second grasping plate **8** and pinch the tissue inserted between the first grasping plate **6** and the second grasping plate **8** from both sides.

The structure of the manipulation member **2** may be appropriately designed as desired. For example, the manipulation member **2** may be formed integrally with the grasping member **1** such that the first grasping plate **6** and the second grasping plate **8** move in cooperation with the manipulation member **2** by manipulating the manipulation member **2.**

More specifically, the manipulation member **2** may have a structure in which two manipulation plates, a manipulation plate **2a** and a manipulation plate **2b** form the manipulation member **2** and are integrally formed with the first grasping plate **6** and the second grasping plate **8,** respectively, and the manipulation member **2** moves such that the first grasping plate **6** and the second grasping plate **8** are overlaid or separated by applying or removing force to or from the manipulation plates **2a** and **2b,** or adjusting the force to the manipulation plates **2a** and **2b**.

The manipulation member **2** may include an adjusting mechanism for applying force in a direction such that the pair of the first grasping plate 6 and the second grasping plate **8** overlay or applying force in a direction such that the first grasping plate **6** and the second grasping plate **8** move back to their original positions. Such an adjusting mechanism may be an elastic body or a spring.

The manipulation member **2** preferably has a structure such that it can give force sufficient for stably grasping a tissue and which does not cause damage to the tissue.

(Wiring Portion or Connection portion **3**)

The wiring portion **3** connects the grasping member **1** or the manipulation member **2** to another tool (for example, an arm of a stabilizer) or manipulation means.

The structure of the wiring portion **3** is not specifically limited. However, a flexible wire having a flexibility which allows the operator to freely bend the wire with one hand is preferable. For example, it may be a wire having a flexible backbone structure. The wire may be subjected to a process such as dying, vinyl-coating or the like as appropriate.

The material of the wiring portion **3** is not specifically limited and can be appropriately set as desired. For example, various types of metals such as iron, aluminum, copper, brass and the like, or various types of plastic may be used.

One end of the wiring portion **3** may be connected to the manipulation member **2.** The structure of the connection point may be appropriately designed. For example, the wiring portion **3** may be connected to the manipulation member **2** via a suitable connection portion **4.** Alternatively, the wiring portion **3** may be integrally formed with the manipulation member **2.**

In the case where the wiring portion **3** is connected to the manipulation member **2** via the connection portion **4**, the structure of the connection portion **4** may be appropriately designed as desired. Particularly, a structure in which the wiring portion **3** can be moved as freely as possible with the connection portion **4** being a fulcrum is preferable. The specific structure of the connection portion **4** may be a structure which includes a rotation axis which moves in cooperation with the wiring portion **3** and a bearing for supporting the rotation axis which is fixed to the manipulation member **2,** and in which wire is connected so as to rotate freely with the rotation axis as a fulcrum.

The structure of the other end of the wiring portion **3** may also be appropriately designed as desired. It may include, for example, a fixing tool **33** for fixing to a rib spreader. The fixing tool may be, for example, a clip.

(A Method for Using the Surgical Holder H)

The surgical holder H of the present invention can be used for grasping or manipulating tissue during surgery.

A tissue to be grasped is not specifically limited. The tissue may be, for example, a tubular tissue or linear tissue, such as a blood vessels, bowel, nerve, etc., and a surrounding tissue thereof. Particularly, the surgical holder H of the present invention is suitable for grasping blood vessel tissues, such as arteries, veins, and the like.

The surgical holder H of the present invention may be preferably used for surgery, especially, blood vessel surgery. Particularly the surgical holder H may be preferably used for grasping various types of blood tissues in any types of bypass surgery. For example, it is possible to grasp and fix almost all types of artery, such as internal mammary artery (IMA) which is used most frequently as an artery graft for coronary bypass surgery, gastroepiploic arteries (GEA), inferior epigastric artery (IEA), radial artery (RA), and the like. It is also possible to grasp and fix veins such as the large saphenous vein (SV).

Furthermore, the surgical holder H of the present invention can grasp tissues of various forms such as, a tissue having both a tubular tissue and a surrounding tissue, a skeletonized tissue, and the like. For example, according to the present invention, it is possible to grasp an artery which is removed with the surrounding tissue as a so-called pedicle. Further, it is possible to grasp the skeletonized artery.

Further, the grasped tissue may be used for manipulating, sequential grafting or the like.

Specifically, the surgical holder H of the present invention may be used as described below.

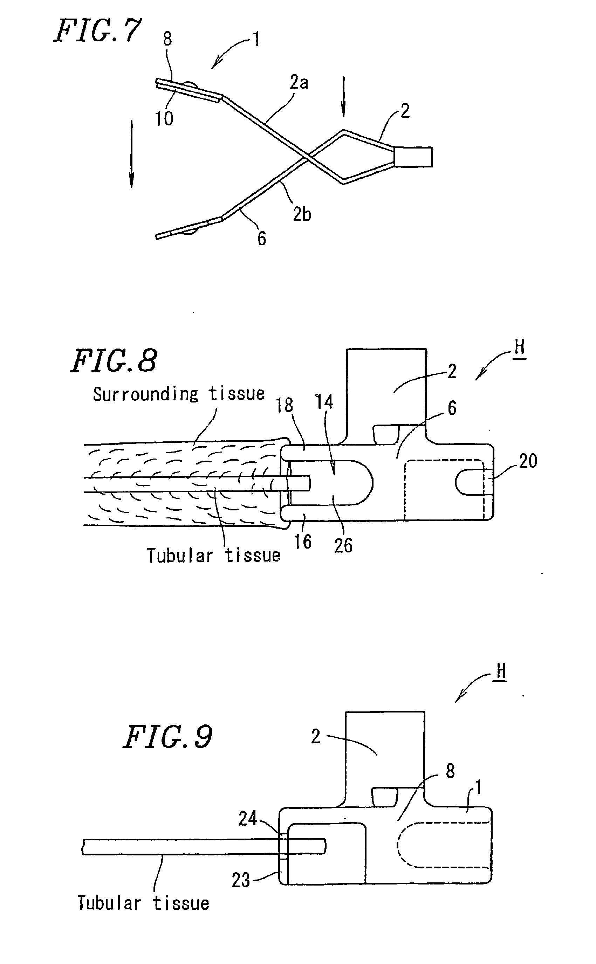

One example is a method of use for grasping a tubular tissue or a surrounding tissue of a linear tissue with the retaining portion **18** for defining a shape of the opening **14** of the first grasping plate 6 and the covering portion **26** of the second grasping plate **8,** as shown in Figure 8. This method can be applied to the case in which, for example, an arterial graft removed with the surrounding tissue is grasped as a pedicle.

The second example is a method of use for grasping a tubular tissue or a linear tissue with the tissue grasping space **15** formed by the recessed portion **20** of the first grasping plate **6** and the curved portion **24** of the second grasping plate **8**, as shown in Figure **9**. This method can be applied to the case in which, for example, a skeletonized artery graft is grasped.

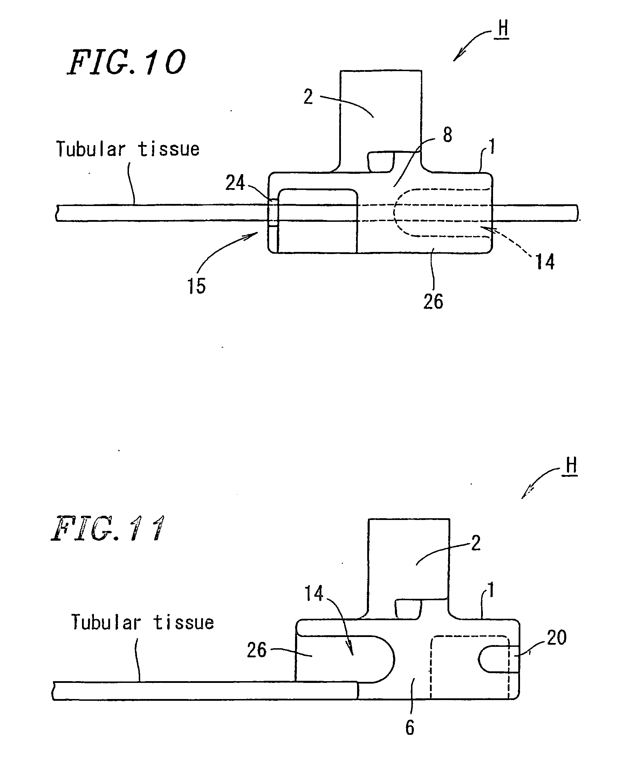

The third example is a method of use for grasping a tubular tissue or a linear tissue at at least two points with the retaining portion **18** of the first grasping plate **6** and the covering portion **26** of the second grasping plate **8** grasping the tubular tissue or the linear tissue at one point, and with the tissue grasping space **15** formed by the recessed portion **20** of the first grasping plate **6** and the curved portion **24** of the second grasping plate **8** grasping the tubular tissue or the linear tissue at another point as shown in Figure **10****.** This method can be applied to the case in which, for example, a skeletonized artery graft is grasped at two points.

The fourth example is a method of use for grasping a tubular tissue by inserting the end portion of the part which defines the shape of the opening **14** into a tube of the tubular tissue, as shown in Figure **11****.** This method can be applied to the case in which, for example, a veinous graft is grasped.

Furthermore, the surgical holder H of the present invention may be used to perform the sequential grafting described below. Herein, cut portion means a portion where the tissue is incised or amputated, and it refers to, for example, an incised portion formed by a midline incision into a side surface of the tubular tissue, and/or a stump portion which locates at the end portion of the tubular tissue.

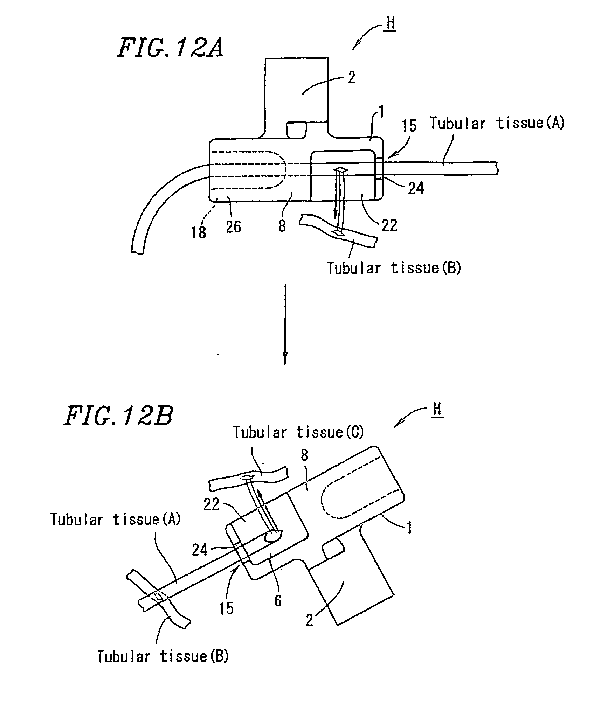

First, as shown in Figure **12A****,** the surgical holder H of the present invention is used to grasp a point of a tubular tissue (A) by the retaining portion 18 of the first grasping plate **6** and the covering portion **26** of the second grasping plate 8 and grasp another point of the tubular tissue (A) by the tissue grasping space **15** formed by the recessed portion **20** of the first grasping plate **6** and the curved portion **24** of the second grasping plate **8** and to perform an anastomosis manipulation of a cut portion of the tubular tissue (A) and a cut portion of a tubular tissue (B) positioned on the supporting portion **22.** At the same time, as shown in Figure **12B****,** the tubular tissue (A) is grasped with the tissue grasping space **15** formed by the recessed portion **20** of the first grasping plate 6 and the curved portion **24** of the second grasping plate **8** to perform an anastomosis manipulation of a cut portion of the tubular tissue (A) and a cut portion of a tubular tissue (C) positioned on the supporting portion **22.**

The way to grasp a tubular tissue and the direction may be appropriately changed in accordance with the progress of the manipulation. Thus, sequential grafting can be efficiently performed. Further, the supporting portion **22** can also be used as a manipulation platform in an anastomosis manipulation.

As described above, the surgical holder H of the present invention is a multifunction tool which can be used for various operations by itself and which significantly improves manipulability or effectiveness during surgery.

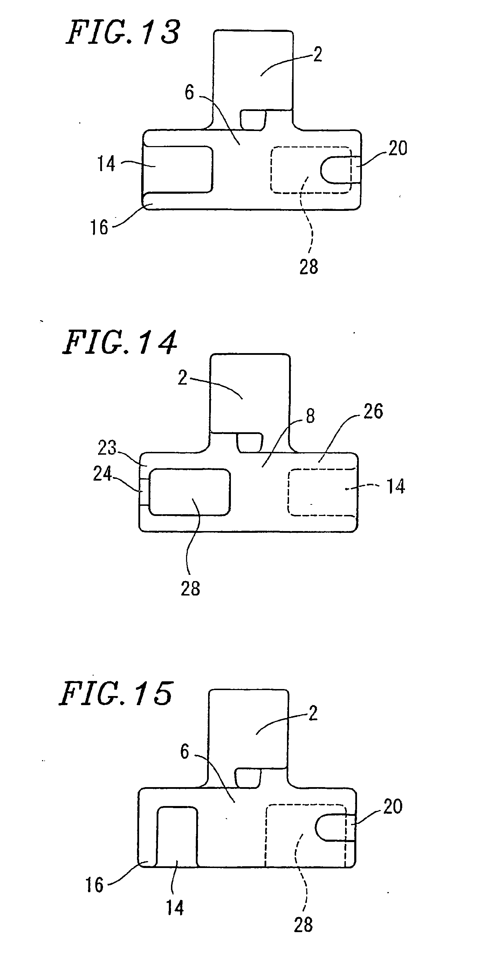

Figures **13** and **14** show a structure of a grasping member **1** according to another embodiment of the present invention.

In this embodiment, a second grasping plate **8** which forms part of the grasping member **1** includes a covering portion **26** formed so as to cover an entire surface or a part of an opening **14** of a first grasping plate **6,** a non-covering portion **28** which does not cover the first grasping plate **6,** and a fixing portion **23** having a curved portion **24.** More specifically, the second grasping plate **8** includes the fixing portion **23** provided to extend from the covering portion **26,** which is formed so as to cover an entire surface or a part of the opening **14** of the first grasping plate **6,** through two elongated pieces, and includes the non-covering portion **28** with an opening of a rectangular shape formed by an edge of the covering portion **26,** the elongated piece, and the fixing portion **23.**

The first grasping plate **6** is formed with the opening **14** having a squared C-shape. Other portions of the structure are the same as those in the above embodiment.

According to the above-described structure, the curved portion **24** is formed in a part of a frame body forming the non-covering portion **28.** The strength of the curved portion **24** can be enhanced.

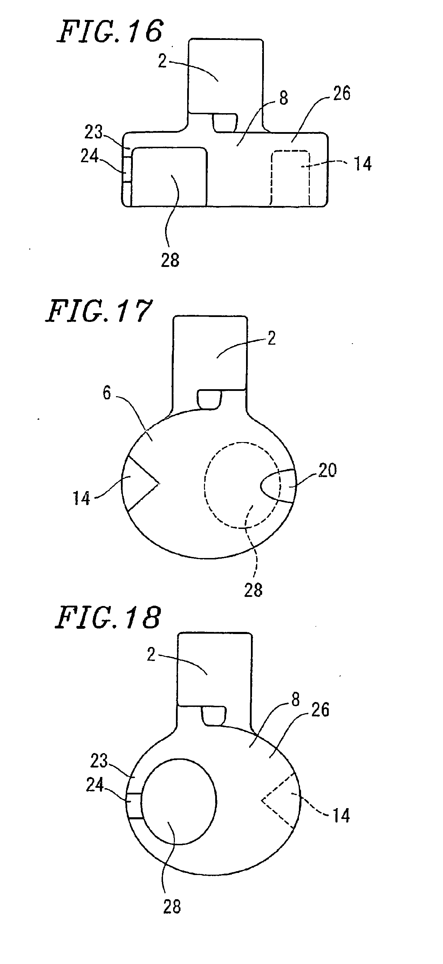

Figures **15** and **16** show a structure of a grasping member **1** according to still another embodiment of the present invention.

In this embodiment, an opening **14** which is opened along a side edge of a first grasping plate 6, which is opposed to a manipulation member **2**, is formed. Other portions of the structure are the same as those in the above embodiment.

Figures **17** and **18** show a structure of a grasping member **1** according to yet another embodiment of the present invention.

In this embodiment, a first grasping plate **6** and a second grasping plate **8** which is located to oppose the first grasping plate **6** are formed into circular or elliptic shapes. An opening **14** formed at one end portion of the first grasping plate **6** is formed into a fan-shape (three-sided shape with one curved shape). A non-covering portion **28** formed in the second grasping plate **8** has a circular or elliptic shape. Other portions of the structure are the same as those in the above embodiment.

According to the above-described embodiment, the strength of the curved portion **24** or the fixing portion **23** forming the curved portion **24** can be enhanced. Further, since there are no corners in the grasping member **1**, there is less fracture or damage.

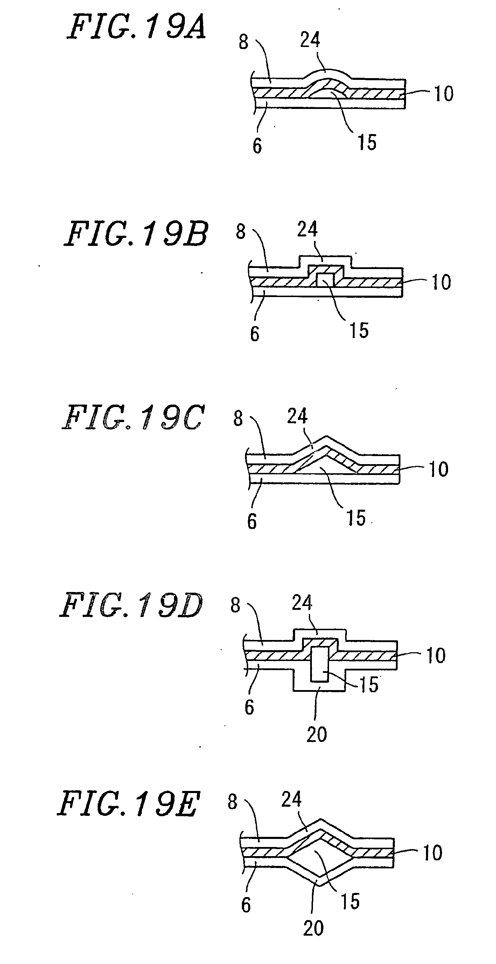

Moreover, the tissue grasping space **15** formed between the first grasping plate **6** and the second grasping plate 8 may have the structure as shown in Figures **19A** through **19E****,** but this does not fall within the scope of the present invention.

Figures **19A** through **19C** show a structure in which a recessed portion or a curved portion is formed on only one of the first grasping plate **6** and the second grasping plate **8.** Figure **19A** shows a structure having a curved recessed portion. Figure **19B** shows a structure with a recessed portion having a rectangular cross-section. Figure **19C** shows a structure with a recessed portion having a triangular cross-section.

Figures **19D** and **19E** show a structure with a recessed portion formed in an inner surface of the first grasping plate **6** and the second grasping plate **8.** Figure **19D** shows a structure formed with a recessed portion having a rectangular cross-section. Figure **19E** shows a structure formed with a recessed portion having a triangular cross-section.

Hereinafter, Examples of the surgical holder H of the present invention will be described with respect to a manner when cardiac surgery is performed, with reference to the drawings. The present invention is not limited to these Examples.

(Examples)

In the Examples, tubular tissues in the drawings are explained as an artery or a vein.

(Example 1: Grasping of a Tissue Including a Tubular Tissue and a Surrounding Tissue)

As shown in Figure **8****,** the surgical holder H of the present invention is used to grasp an artery removed with the surrounding tissue as a so-called pedicle. A tissue including a tubular tissue and a surrounding tissue thereof is inserted between the first grasping plate **6** and the second grasping plate **8** from the side of the opening **14.** The manipulation portion **2** manipulates the first grasping plate **6** and the second grasping plate 8 so as to overlay and grasp the tissue. At this time, the edge portion 16 of the retaining portion **18** which defines the shape of the opening **14** of the first grasping plate **6** and the covering portion **26** of the second grasping plate **8** grasps the surrounding tissue.

The covering portion **26** of the second grasping plate **8** supports a tubular tissue positioned at the opening **14**. As described above, by grasping the surrounding tissue of the tubular tissue not the tubular tissue, it is possible to retain the tissue without causing damage to the tubular tissue.

(Example 2: Grasping of a Skeletonized Tissue)

As shown in Figure **9**, the surgical holder H of the present invention is used to grasp a skeletonized tissue. The recessed portion **20** of the first grasping plate **6** or the curved portion **24** of the second grasping plate **8** positions an artery. The manipulation member **2** manipulates the first grasping plate **6** and the second grasping plate **8** so as to overlay to fix the tissue. Thus, it is possible to stably hold the skeletonized tissue without cutting it.

(Example 3: Grasping of a Skeletonized Tubular Tissue at a Plurality of Points)

As shown in Figure **10**, the surgical holder H of the present invention is used such that, with one point of a skeletonized artery positioned at the opening **14** of the first grasping plate **6** and another point of the tubular tissue positioned at the tissue grasping space **15** formed by the recessed portion **20** of the first grasping plate **6** and the curved portion **24** of the second grasping plate **8,** the manipulation member **2** manipulates the first grasping plate **6** and the second grasping plate **8** so as to overlay. The retaining portion **18** of the first grasping plate **6** and the covering portion **26** of the second grasping plate **8** grasp the tubular tissue at one point and the tissue grasping space **15** formed by the recessed portion **20** of the first grasping plate 6 and the curved portion **24** of the second grasping plate **8** grasps another point of the tissue. Thus, the tissue is grasped at at least two points. By grasping the tissue at a plurality of points as described above, it is possible to stably grasp the tissue.

(Example **4**: Grasping of a Vein)

As shown in Figure **11**, the surgical holder H of the present invention is used to grasp a vein. The end portion of the part which defines a shape of the opening **14** of the first grasping plate **6** is inserted into a tube of a vein and the vein hangs suspended and is grasped. A side surface of the blood vessel can be further fixed by the covering portion **26** of the second plate **8** to grasp the vein more stably.

(Example 5: Sequential Grafting)

The surgical holder H of the present invention is used to grasp a skeletonized artery (A) by the retaining portion **18** of the first grasping plate 6 and the covering portion **26** of the second grasping plate **8** at one point and by the tissue grasping space **15** formed by the recessed portion **20** of the first grasping plate 6 and the curved portion **24** of the second grasping plate **8** at another point such that the tissue is grasped at at least two points. A side surface of the artery (A) which is positioned between the two points is incised in a direction parallel to a longitudinal direction of the blood vessel. The incised portion of the artery (A) is positioned on the supporting portion **22** of the first grasping plate.

Then, manipulation for side-to-side anastomosis is performed between an incised portion of an artery (B) and the incised portion of the artery (A) (Figure **12A**). Parachute suture is performed on both blood vessels with just a few stitches. Next, the artery (A) is removed from the grasping member **1** and adhered to the anastomotic portion with the artery (B). Then, the rest of the suturing is performed to complete the side-to-side anastomosis.

Subsequently, the surgical holder H of the present invention is used to grasp the proximate end of the artery (A) with the tissue grasping space **15** formed by the recessed portion **20** of the first grasping plate **6** and the curved portion **24** of the second grasping plate **8** and position the stump of the artery (A) on the supporting portion of the first grasping plate **6**. Then, the manipulation for end-to-side anastomosis is performed between an incised portion of an artery (C) and the stump of the artery (A) (Figure **12B**). Parachute suture is performed on both blood vessels with just a few stitches. Next, the artery (A) is removed from the grasping plate and adhered to the anastomotic portion with the artery (C). Then, the rest of the suturing is performed to complete the end-to-side anastomosis.

INDUSTRIAL APPLICABILITY

As described above, the surgical holder of the present invention can address various types of manipulations of surgical operations, and it is preferable for use in blood vessel surgery, and more particularly, cardiac blood vessel surgery.

## Claims

1. A surgical holder comprising a grasping member (1) for grasping a tissue, a manipulation member (2) for manipulating the grasping member (1), and a connection portion (3) with one end connected to the manipulation member (2), wherein:
the grasping member (1) includes a first grasping plate (6) and a second grasping plate(8) provided so as to oppose the first grasping plate (6) in a movable manner so that they are able to become closer to each other or more distanced from each other;
the first grasping plate (6) includes a retaining portion (18) having an opening (14) which is opened toward an outer side of the first grasping plate (6) and a supporting portion (22) having a recessed portion (20), the opening (14) being provided in one end of the first grasping plate (6) and the recessed portion (20) being provided in the other end of the first grasping plate (6),
the second grasping plate (8) includes a covering portion (26) formed so as to cover an entire surface or a part of the opening of the first grasping plate (6), a non-covering portion (28) which does not cover the first grasping plate (6), and a fixing portion (23) having a curved portion (24), the covering portion (26) being provided in one end of the second grasping plate (8) and the fixing portion (23) being provided in the other end of the second grasping plate (8),
the curved portion (24) opposing the recessed portion (20) to form a generally tubular tissue grasping space (15) when the first grasping plate (6) and the second grasping plate (8) are positioned so as to oppose one another,
the grasping member further including a first grasping portion which can grasp a first part of the tissue between the retaining portion (18) of the first grasping plate (6) and the covering portion (26) of the second grasping plate (8) in one end portion of the grasping member (1), and the opening (14) exposing another part of the tissue when the first part of the tissue is grasped by the first grasping portion; and
a second grasping portion which can form the generally tubular tissue grasping space (15) between the recessed portion (20) of the first grasping plate (6) and the curved portion (24) of the second grasping plate (8) in another end portion of the grasping member (1).

2. A surgical holder according to claim 1, wherein a tissue protection material (10) is attached to an opposing surface of the first grasping plate (6) and/or the second grasping plate (8).

3. A surgical holder according to claim 2, wherein the tissue protection material (10) is permeated with medicines.

4. A surgical holder of claim 1 wherein:
the grasping member is formed into a rectangular shape with the manipulation member elongated from a side thereof.

5. A surgical holder of claim 1 wherein:
the opening (14) is of a U-shape or substantially a U-shape.

6. A surgical holder according to claim 5, wherein the tissue to be grasped is a tubular tissue with a surrounding tissue, and the surrounding tissue is grasped by a part which defines the shape of the opening (14) of the first grasping plate (6) and the covering portion (26) of the second grasping plate (8).

7. A surgical holder according to claim 5, wherein the tissue to be grasped is a tubular tissue, and the tubular tissue is grasped by a tissue grasping space (15) formed by the recessed portion (20) of the first grasping plate (6) and the curved portion (24) of the second grasping plate (8).

8. A surgical holder according to claim 5, wherein the tissue to be grasped is a tubular tissue, and the tubular tissue is grasped with one point of the tubular tissue being grasped by the retaining portion (18) of the first grasping plate (6) and the covering portion (26) of the second grasping plate (8), and another point being grasped by a tissue grasping space (15) formed by the recessed portion (20)of the first grasping plate (6) and the curved portion (24) of the second grasping plate (8).

9. A surgical holder according to claim 5, wherein the tissue to be grasped is a tubular tissue, and an end portion of the retaining portion (18) which defines the opening (14) of the first grasping plate (6) is inserted into a tube of the tubular tissue to grasp the tubular tissue.

10. A surgical holder according to claim 5, wherein the tissue to be grasped is a tubular tissue (A), and, (i) with one point of the tubular tissue (A) grasped by the retaining portion (18) of the first grasping plate (6) and the covering portion (26) of the second grasping plate (8) and another point of the tubular tissue (A) grasped by the tissue grasping space (15) formed by the recessed portion (20) of the first grasping plate (6) and the curved portion (24) of the second grasping plate (8), anastomosis manipulation between a cut portion of the tubular tissue (A) and a cut portion of a tubular tissue (B) positioned on the supporting portion (22) can be performed, and then, (ii) with the tubular tissue (A) grasped by the tissue grasping space (15) formed by the recessed portion (20) of the first grasping plate (6) and the curved portion (24) of the second grasping plate (8), anastomosis manipulation between a cut portion of the tubular tissue (A) and a cut portion of the tubular tissue (C) positioned on the supporting portion (22) can be performed.

11. A surgical holder according to claim 5, wherein the retaining portion (18) includes an edge portion (16) having two elongated end portions, the edge portion (16) forming the peripheral portion of the opening (14) to define the shape of the opening (14), and the end portion of the edge portion (14) having a length and being configured to be inserted into a tube of a tubular tissue for grasping the tubular tissue.

12. A surgical holder according to claim 1, wherein the tissue is a tubular tissue.

13. A surgical holder according to claim 12, wherein the tubular tissue is a blood vessel.

## Patentansprüche

1. Chirurgische Haltervorrichtung mit einem Greifglied (1) zum Ergreifen von Gewebe, einem Manipulations-Glied (2) zum Manipulieren des Greifgliedes (1) und einem Verbindungsbereich (3) mit einem Ende, das mit dem Manipulations-Glied (2) gekoppelt ist, wobei
das Greifglied (1) eine erste Greifplatte (6) und eine zweite Greifplatte (8) enthält, die so vorgesehen bzw. angeordnet ist, dass sie der ersten Greifplatte (6) auf bewegliche Weise gegenüberliegt, so dass sie näher zueinander oder in einen größere Abstand voneinander kommen können;
die erste Greifplatte (6) enthält einen Bewahrungs- bzw. Rückhalte-Bereich (18) mit einer Öffnung (14), die zu einer äußeren Seite der ersten Greifplatte (6) hin geöffnet ist, und mit einem Halte- bzw. Tragbereich (22) mit seinem ausgesparten Bereich (20), wobei die Öffnung (14) in einem Ende der ersten Greifplatte (6) und der ausgesparte Bereich (20) in dem anderen Ende der ersten Greifplatte (6) vorgesehen sind;
die zweite Greifplatte (8) enthält einen Abdeckbereich (26), der so ausgebildet ist, dass er die gesamte Oberfläche oder einen Teil der Öffnung der ersten Greifplatte (6) bedeckt, einen Nicht-Abdeckungsbereich (28), der die erste Greifplatte (6) nicht bedeckt, und einen Befestigungsbereich (23) mit einem gekrümmten Bereich (24), wobei der Abdeckbereich (26) in einen Ende der zweiten Greifplatte (8) und der Bestigungsbereich (23) in dem anderen Ende der zweiten Greifplatte (8) vorgesehen sind,
wobei der gekrümmte Bereich (24) dem ausgesparten Bereich (20) gegenüberliegt, um einen im allegemeinen rohrförmigen, das Gewebe ergreifenden Raum (15) zu bilden, wenn die erste Greifplatte (6) und die zweite Greifplatte (8) so angeordnet sind, dass sie einander gegenüberliegen,
wobei das Greifelement weiterhin enthält: Einen ersten Greifbereich, der einen ersten Teil des Gewebes zwischen dem Rückhalte-Bereich (18) der ersten Greifplatte (6) und dem Abdeckbereich (26) der zweiten Greifplatte (8) in einem Endbereich des Greifgliedes (1) ergreifen kann und die Öffnung (14) einen weiteren Teil des Gewebes freilegt, wenn der erste Teil des Gewebes durch den ersten Greifbereich erfasst wird; und
einen zweiten Greifbereich, der den im allegemeinen rohrförmigen, das Gewebe ergreifenden Raum (15) zwischen dem ausgesparten Bereich (20) der ersten Greifplatte (6) und dem gekrümmten Bereich (24) der zweiten Greifplatte (8) in einem weiteren Endbereich des Greifgliedes bilden kann.

2. Chirurgische Haltervorrichtung nach Anspruch 1, wobei ein Gewebe-Protektionsmaterial (10) an einer gegenüberliegenden Oberfläche der ersten Greifplatte (6) und/oder der zweiten Greifplatte (8) angebracht ist.

3. Chirurgische Haltervorrichtung nach Anspruch 2, wobei das Gewebe-Protektionsmaterial (10) von Medikamenten durchdrungen ist.

4. Chirurgische Haltervorrichtung nach Anspruch 1, wobei das Greifglied in einer rechteckigen Form gestaltet ist, wobei das Manipulations-Glied von einer Seite hiervon verlängert ist.

5. Chirurgische Haltervorrichtung nach Anspruch 1, wobei die Öffnung (14) eine U-Form oder im wesentlichen eine U-Form hat.

6. Chirurgische Haltervorrichtung nach Anspruch 5, wobei das zu ergreifende Gewebe ein rohrförmiges Gewebe mit einem umgebenden Gewebe ist und das umgebende Gewebe durch einen Teil erfasst wird, der die Form der Öffnung (14) der ersten Greifplatte (6) und des Abdeckbereichs (26) der zweiten Greifplatte (8) definiert.

7. Chirurgische Haltervorrichtung nach Anspruch 5, wobei das zu ergreifende Gewebe ein rohrförmiges Gewebe ist und das rohrförmige Gewebe durch einen das Gewebe ergreifenden Raum (15) erfasst wird, der durch den ausgesparten Bereich (20) der ersten Greifplatte (6) und den gekrümmten Bereich (24) der zweiten Greifplatte (8) gebildet wird.

8. Chirurgische Haltervorrichtung nach Anspruch 5, wobei das zu ergreifende Gewebe ein rohrförmiges Gewebe ist und das rohrförmige Gewebe erfasst wird, indem ein Punkt des rohrförmigen Gewebes durch den Rückhalte-Bereich (18) der ersten Greifplatte (6) und den Abdeckbereich (26) der zweiten Greifplatte (8) erfasst wird, und ein weiterer Punkt durch einen das Gewebe ergreifenden Raum (15) erfasst wird, der durch den ausgesparten Bereich (20) der ersten Greifplatte (6) und den gekrümmte Bereich (24) der zweiten Greifplatte (8) gebildet wird.

9. Chirurgische Haltervorrichtung nach Anspruch 5, wobei das zu ergreifende Gewebe ein rohrförmiges Gewebe ist und ein Endbereich des Rückhaltebereichs (18), der die Öffnung (14) der ersten Greifplatte (6) definiert, in ein Rohr des rohrförmige Gewebes eingeführt wird, um das rohrförmige Gewebe zu erfassen.

10. Chirurgische Haltervorrichtung nach Anspruch 5, wobei das zu ergreifende Gewebe ein rohrförmiges Gewebe (A) ist, und
(i) wenn ein Punkt des rohrförmige Gewebes (A) durch den Zurückhaltebereich (18) der ersten Greifplatte (6) und den Abdeckbereich (26) der zweiten Greifplatte (8) und ein weiterer Punkt des rohrförmigen Gewebes (A) durch den Gewebe ergreifenden Raum (15) erfasst werden, der durch den ausgesparten Bereich (20) der ersten Greifplatte (6) und den gekrümmten Bereich (24) der zweiten Greifplatte (8) gebildet wird, eine Anastomose-Manipulation zwischen einem geschnittenen Bereich des rohrförmigen Gewebes (A) und einem geschnittenen Bereich des rohrförmigen Gewebes (B), die auf dem Tragbereich (22) positioniert sind, durchgeführt werden kann, und dann
(ii) eine Anastomose-Manipulation zwischen einem geschnittenen Bereich des rohrförmigen Gewebes (A) und einem geschnittenen Bereich des rohrförmigen Gewebes (C), das auf dem Tragbereich (22) positioniert ist, durchgeführt werden kann, wenn das rohrförmige Gewebe (A) durch den das Gewebe ergreifenden Raum (15) erfasst wird, der durch den ausgesparten Bereich (20) der ersten Greifplatte (6) und den gekrümmten Bereich (24) der zweiten Greifplatte (8) ergriffen wird.

11. Chirurgische Haltervorrichtung nach Anspruch 5, wobei der Zurückhalte-Bereich (18) einen Randbereich (16) mit zwei langgestreckten Endbereichen enthält, wobei der Randbereich (16) den Umfangsbereich der Öffnung (14) bildet, um die Form der Öffnung (14) zu definieren, und die Endbereiche des Randbereichs (14) eine solche Länge haben und so konfiguriert sind, um in ein Rohr eines rohrförmigen Gewebes zum Erfassen des Gewebes eingeführt zu werden.

12. Chirurgische Haltervorrichtung nach Anspruch 1, wobei das Gewebe ein rohrförmiges Gewebe ist.

13. Chirurgische Haltervorrichtung nach Anspruch 12, wobei das rohrförmige Gewebe ein Blutgefäß ist.

## Revendications

1. Dispositif chirurgical de maintien comprenant un élément de préhension (1) destiné à saisir un tissu, un élément de manipulation (2) destiné à manipuler l'élément de préhension (1), et une partie de raccordement (3) avec une extrémité reliée à l'élément de manipulation (2), où :
l'élément de préhension (1) comporte une première plaque de préhension (6) et une deuxième plaque de préhension (8) prévue pour s'opposer à la première plaque de préhension (6) de manière mobile de sorte qu'elles puissent se rapprocher ou s'éloigner l'une de l'autre ; la première plaque de préhension (6) comporte une partie de retenue (18) ayant une ouverture (14) qui est ouverte vers un côté extérieur de la première plaque de préhension (6) et une partie de support (22) ayant une partie évidée (20), l'ouverture (14) étant prévue dans une extrémité de la première plaque de préhension (6) et la partie évidée (20) étant prévue dans l'autre extrémité de la première plaque de préhension (6),
la deuxième plaque de préhension (8) comporte une partie couvrante (26) formée de manière à couvrir toute la surface ou une partie de l'ouverture de la première plaque de préhension (6), une partie non-couvrante (28) qui ne couvre pas la première plaque de préhension (6), et une partie de fixation (23) ayant une partie incurvée (24), la partie couvrante (26) étant prévue dans une extrémité de la deuxième plaque de préhension (8) et la partie de fixation (23) étant prévue dans l'autre extrémité de la deuxième plaque de préhension (8),
la partie incurvée (24) s'opposant à la partie évidée (20) pour former un espace généralement tubulaire (15) de préhension du tissu lorsque la première plaque de préhension (6) et la deuxième plaque de préhension (8) sont positionnées de manière à s'opposer l'une à l'autre,
les éléments de préhension comportant en outre une première partie de préhension qui peut saisir une première partie du tissu entre la partie de retenue (18) de la première plaque de préhension (6) et la partie couvrante (26) de la deuxième plaque de préhension (8) dans une partie d'extrémité de l'élément de préhension (1), et l'ouverture (14) exposant une autre partie du tissu lorsque la première partie est saisie par la première partie de préhension ; et
une deuxième partie de préhension qui peut former l'espace généralement tubulaire (15) de préhension du tissu entre la partie évidée (20) de la première plaque de préhension (6) et la partie incurvée (24) de la deuxième plaque de préhension (8) dans une autre partie d'extrémité de l'élément de préhension (1).

2. Dispositif chirurgical de maintien selon la revendication 1, dans lequel un matériau (10) de protection du tissu est fixé à une surface opposée de la première plaque de préhension (6) et/ou de la deuxième plaque de préhension (8).

3. Dispositif chirurgical de maintien selon la revendication 2, dans lequel le matériau (10) de protection du tissu est imprégné de médicaments.

4. Dispositif chirurgical de maintien selon la revendication 1 dans lequel :
l'élément de préhension a une forme rectangulaire avec l'élément de manipulation allongé à partir d'un côté de celui-ci.

5. Dispositif chirurgical de maintien selon la revendication 1 dans lequel :
l'ouverture (14) a une forme en U ou sensiblement une forme en U.

6. Dispositif chirurgical de maintien selon la revendication 5, dans lequel le tissu à saisir est un tissu tubulaire avec un tissu environnant, et le tissu environnant est saisi par une partie qui définit la forme de l'ouverture (14) de la première plaque de préhension (6) et la partie couvrante (26) de la deuxième plaque de préhension (8).

7. Dispositif chiruraical de maintien selon la revendication 5, dans lequel le tissu à saisir est un tissu tubulaire, et le tissu tubulaire est saisi par un espace (15) de préhension du tissu formé par la partie évidée (20) de la première plaque de préhension (6) et la partie incurvée (24) de la deuxième plaque de préhension (8).

8. Dispositif chirurgical de maintien selon la revendication 5, dans lequel le tissu à saisir est un tissu tubulaire, et le tissu tubulaire est saisi avec un point du tissu tubulaire qui est saisi par la partie de retenue (18) de la première plaque de préhension (6) et la partie couvrante (26) de la deuxième plaque de préhension (8), et un autre point étant saisi par un espace (15) de préhension du tissu formé par la partie évidée (20) de la première plaque de préhension (6) et la partie incurvée (24) de la deuxième plaque de préhension (8).

9. Dispositif chirurgical de maintien selon la revendication 5, dans lequel le tissu à saisir est un tissu tubulaire, et une partie d'extrémité de la partie de retenue (18) qui définit l'ouverture (14) de la première plaque de préhension (6) est insérée dans un tube du tissu tubulaire pour saisir le tissu tubulaire.

10. Dispositif chirurgical de maintien selon la revendication 5, dans lequel le tissu à saisir est un tissu tubulaire (A), et, (i) avec un point du tissu tubulaire (A) saisi par la partie de retenue (18) de la première plaque de préhension (6) et la partie couvrante (26) de la deuxième plaque de préhension (8) et un autre point du tissu tubulaire (A) saisi par l'espace (15) de préhension du tissu formé par la partie évidée (20) de la première plaque de préhension (6) et la partie incurvée (24) de la deuxième plaque de préhension (8), une manipulation d'anastomose entre une partie découpée du tissu tubulaire (A) et une partie découpée d'un tissu tubulaire (B) positionnée sur le partie de support (22) peut être effectuée, et ensuite, (ii) avec le tissu tubulaire (A) saisi par l'espace (15) de préhension du tissu formé par la partie évidée (20) de la première plaque de préhension (6) et la partie incurvée (24) de la deuxième plaque de préhension (8), une manipulation d'anastomose entre une partie découpée du tissu tubulaire (A) et une partie découpée du tissu tubulaire (C) positionnée sur la partie de support (22) peut être effectuée.

11. Dispositif chirurgical de maintien selon la revendication 5, dans lequel la partie de retenue (18) comporte une partie de bordure (16) ayant deux parties d'extrémité allongées, la partie de bordure (16) formant la partie périphérique de l'ouverture (14) pour définir la forme de l'ouverture (14), et les parties d'extrémité de la partie de bordure (14) ayant une longueur et étant configurées pour être insérées dans un tube d'un tissu tubulaire pour saisir le tissu tubulaire.

12. Dispositif chirurgical de maintien selon la revendication 1, dans lequel le tissu est un tissu tubulaire.

13. Dispositif chirurgical de maintien selon la revendication 12, dans lequel le tissu tubulaire est un vaisseau sanguin.
